# EUROPEAN PATENT APPLICATION

(11) **EP 2 623 018 A1**
(43) Date of publication of application: **07.08.2013**
(21) Application number: 13153752.4
(22) Date of filing: 01.02.2013
(51) Int. Cl.: A61B 1/00, A61B 1/317, G02B 23/24

(54) **Sheathless arthoscope and system**

(30) Priority: 03.02.2012 US 201261594804 P
(71) Applicant: Arthrex, Inc., Naples, FL 34108-1945 (US)
(72) Inventor: Schmieding, Reinhold, Naples, FL Florida 34108 (US); Kennedy, Bruce, Santa Barbara, CA California 93105 (US)
(74) Representative: Carpmael, Robert Maurice Charles

(57) **Abstract**

A reinforcement system (reinforcement mechanism) for an endoscope or similar instrument. The reinforcement system utilizes coated reinforcement members that extend along a portion of the needle section of the arthroscope, but not along the distal optic portion of the arthroscope, providing a reinforced arthroscopic needle. The reinforcement system includes at least one of first and second reinforcement members along the arthroscopic needle, one of the first and second reinforcement members being fluted to enable fluid transport. The reinforcement system may include reusable (re-sterilizable) components or single-use only components.

## Description

### FIELD OF THE INVENTION

The present invention relates to surgical devices and procedures and, more specifically, to sheathless arthroscopes and methods of use with cannulas during arthroscopic surgery.

### BACKGROUND OF THE INVENTION

Arthroscopes (scopes) are employed to visually inspect a joint, such as a knee or shoulder joint, and to conduct diagnostic viewing of tissue or cartilage within the joint. Arthroscopes typically include a lens system for visualizing the affected area, as well as a fiber optic passage for transmitting light for illuminating the desired area.

In conventional arthroscopic surgical methods, arthroscopes are inserted into sheaths and the arthroscope/sheath system is used together. The sheath tube, which is larger in diameter than the arthroscope, provides protection for the fragile optics within the arthroscope and provides an annular channel for in-flow and/or out-flows of fluid during the procedure. The sheath also typically includes stop-cocks to allow the surgeon to manually control the flow of fluid. A disadvantage of using the sheath is the larger diameter of the sheath (typically 6mm compared with a 4mm arthroscope) which makes the arthroscope/sheath system more difficult to manipulate in tight joints. As a result, some anatomy may be more difficult to visualize.

U.S. Patent No. 8,226,548 describes an arthroscope with radial rib extensions running the full length of the arthroscope needle, making it more difficult to manipulate in the joint. In addition, the arthroscope is intended to be used with a dedicated, larger diameter cannula as opposed to a typical operative cannula. The larger diameter cannula, unlike the operative cannula, extends the full length of the scope and makes the combined system more difficult to manipulate in the joint.

There is a need for an arthroscope with sufficient mechanical strength such that a sheath is not needed. Also needed are cost effective mechanisms that can attach to a conventional instrument (endoscope/arthroscope) and have designs that allow a smaller diameter instrument and manipulation in tight joints. An ergonomic method for surgeons to employ an endoscope/arthroscope without a sheath and with just an operative cannula is also needed.

### SUMMARY OF THE INVENTION

The invention provides an arthroscope designed and constructed with additional structural support along its length to stand up to normal stresses without a sheath. The sheathless arthroscope of the invention reduces surgical times by expediting portal changes and eliminating or minimizing distention loss. At least one reinforcement member extends along a portion of the needle section of the arthroscope, but not along the distal portion of the arthroscope. The resulting arthroscope is smaller in diameter and of sufficient strength to eliminate the need for a sheath.

The present invention also provides an arthroscopy reinforcement system including at least one of first and second reinforcement members provided along the arthroscopic needle (one of the first and second reinforcement members being fluted) to enable fluid transport. The reinforcement system may include reusable (re-sterilizable) components or single-use only components.

The present invention also provides a method of using a sheathless reinforced arthroscope for arthroscopic surgery by *inter alia* inserting the reinforced arthroscope into a cannula (such as an operative cannula) without employing a sheath.

Other features and advantages of the present invention will become apparent from the following description of the invention which refers to the accompany drawings.

### BRIEF DESCRIPTION OF THE DRAWINGS

FIG. illustrates a perspective schematic view of a sheathless arthroscope in accordance with an exemplary embodiment of the present invention.

FIG. 2 illustrates another view of the sheathless arthroscope of FIG. 1.

FIG. 3 illustrates a front schematic view of the sheathless arthroscope of FIG. 1.

FIG. 4 illustrates a top schematic view of the sheathless arthroscope of FIG. 1.

FIG. 5 illustrates a schematic view of the sheathless arthroscope of FIG. 3, rotated about 180 degrees.

FIG. 6 illustrates a bottom schematic view of the sheathless arthroscope of FIG. 1.

FIG. 7 illustrates a perspective view of an exemplary cannula for use with the sheathless arthroscope of the present invention.

FIG. 8 illustrates a side perspective view of the cannula of FIG. 7.

FIG. 9 illustrates a perspective view of the arthroscope of FIG. 2 inserted into the cannula of FIG. 7.

### DESCRIPTION OF THE INVENTION

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the present invention.

The invention provides sheathless arthroscopes (or similar instruments) that are mechanically reinforced with a reinforcement system (mechanism) having at least one reinforcement member extending along a portion of the needle section of the arthroscope (instrument). The reinforced sheathless arthroscopes of the invention reduce surgical times by expediting portal changes and eliminating/minimizing distention loss and the need of external sheaths.

In an exemplary-only embodiment, the reinforcement system (reinforcement mechanism) comprises a reinforcement device (member) which can be attached to (and optionally detached from) the instrument (for example, arthroscope) just distally to a means for introducing illumination (for example, a light port or light post located at the proximal end of the instrument), and at the most proximal portion of the arthroscopic "needle." The reinforcement device (member) is a fluted tubular member which may have an outer diameter of about 6 mm, preferably about 6.2 mm, and extends in a direction parallel to a longitudinal axis of the instrument.

In yet another exemplary-only embodiment, the reinforcement system (reinforcement mechanism) comprises a reinforcement device (member) which can be attached to (and optionally detached from) the instrument, and is located on the arthroscopic "needle" except the most distal area of the needle, providing mechanical support for all but the most distal area of the instrument (scope) which is typically not loaded during surgery. The reinforcement device (member) is a tubular member which may have an outer diameter of about 5 mm, preferably about 5.2 mm, and extends in a direction parallel to a longitudinal axis of the instrument.

In additional embodiments, the reinforcement system (reinforcement mechanism) comprises first and second reinforcement devices (first and second members). The first reinforcement device (first member) is a fluted tubular member having an outer diameter of about 6 mm, preferably about 6.2 mm, which is attached to the instrument just distally to a means for introducing illumination (for example, a light port or light post) and at the most proximal portion of the arthroscopic "needle." The second reinforcement device (second member) is a tubular member having an outer diameter of about 5 mm, preferably about 5.2 mm, which is attached on the length of the arthroscopic "needle" except the most distal area of the needle. The first and second reinforcement devices extend in a direction about parallel to a longitudinal axis of the instrument and provide sufficient mechanical strength to an arthroscope (or similar instrument) such that a sheath is not needed. The first and second reinforcement devices are cost-effective devices that can attach to a conventional sheathless instrument (endoscope/arthroscope) and have a design that allow a smaller diameter for the instrument (arthroscope) and improved manipulation in tight joints (without an external sheath and with just an operative cannula).

The reinforcement system includes elements (members) which may slide onto the shaft of the instrument (endoscope/arthroscope) for attachment to the instrument (endoscope/arthroscope). The elements of the reinforcement system may be also provided as reusable (re-sterilizable devices) or as single-use only devices or as combination of as reusable and single-use devices.

Although the embodiments below will be explained and detailed with reference to a reinforcement system (reinforcement attachment) for a specific instrument, i.e., to a specific sheathless arthroscope, the invention is not limited to this exemplary-only embodiment and has applicability to other similar sheathless endoscopic instruments such as, for example, videoarthroscopes, laparoscopic devices, colonoscopes, gastroscopes, etc., i.e., to sheathless instruments similar to endoscopes/arthroscopes that require insertion of the shaft of the instrument during surgery or surgical intervention, and that permit attachment of the reinforcement devices of the present invention to the shaft of the instrument.

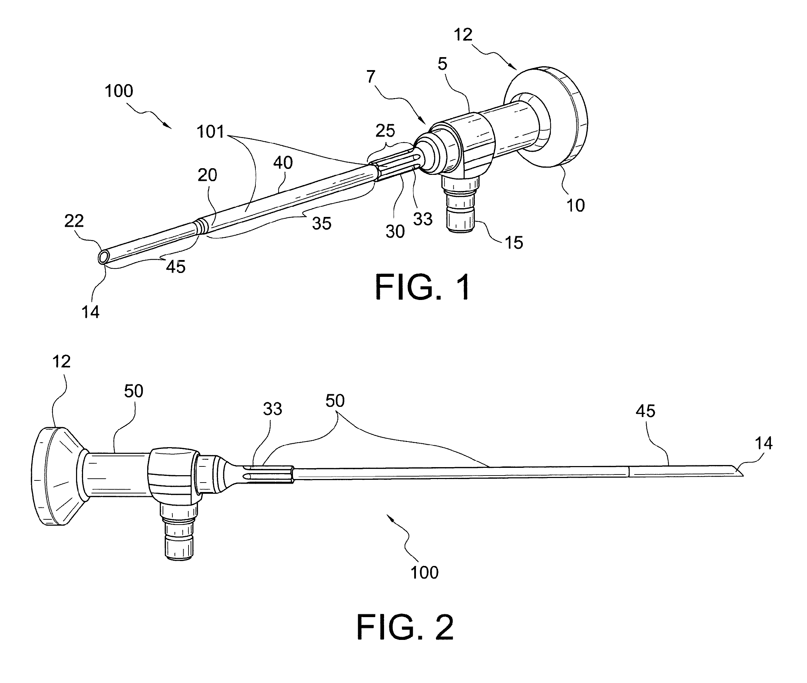

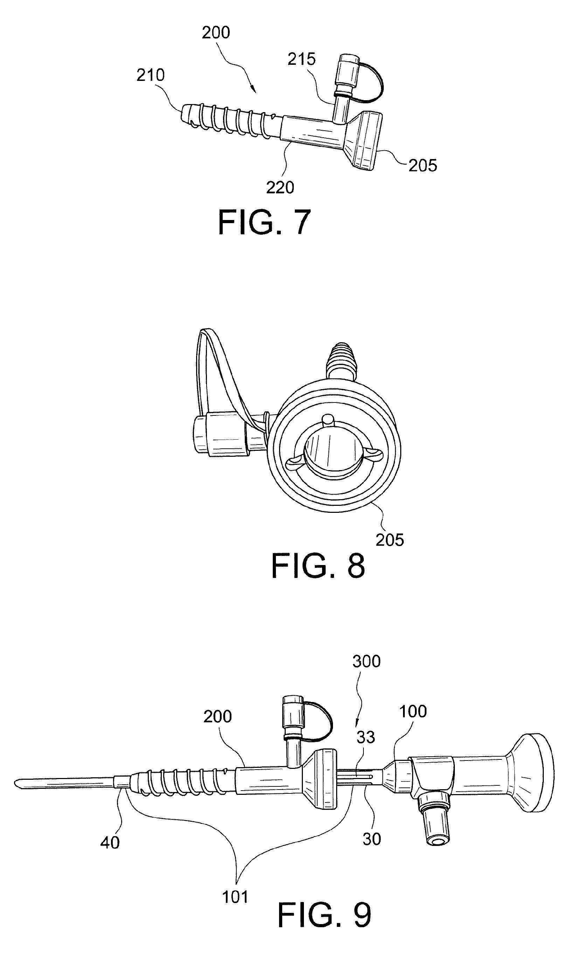

Referring now to the drawings, where like elements are designated by like reference numerals, FIGS. 1-7 illustrate an embodiment of sheathless arthroscope 100 of the present invention provided with reinforcement system 101 (reinforcing system 101) and which can be used with cannulas such as the cannula of described in U.S. Patent Application 2003/0153926, the disclosure of which is incorporated by reference herein in its entirety, or cannula 200 (FIGS. 7-9) to form integrated sheathless arthroscopic system 300 (FIG. 9). The arthroscope 100 is designed, dimensioned and configured to be inserted in a cannula, without a sheath, to be securely positioned within a knee joint.

Arthroscope 100 is preferably manufactured from stainless steel and includes a proximal end 12, a distal end 14 and a body 5. Arthroscopic shaft 20 (needle 20) is a tubular shaft that extends along axis 60 of the instrument from distal side 7 of body 5 to distal end 14 terminating in an angled tip 22 having angles of 0° up to 70°.

Tubular shaft 20 is a hollow, stainless steel tube having a diameter of at least about 4mm, housing all optical and illumination components used in basic endoscopic visualization. Tubular shaft 20 houses a lens system that transmits the image from an objective lens to the viewer (surgeon) typically in the form of a relay lens system (in the case of a rigid instrument) or a bundle of fiberoptics (in the case of a fiberscope). Tubular shaft 20 may also include a high resolution imager (e.g., a CCD imager) mounted at the distal end. Tubular shaft 20 may also house at least one working channel (for example, one or more channels for the introduction of instruments, gases or liquids) that extend longitudinally throughout the shaft 20.

Arthroscope 100 of FIGS. 1-6 and 9 is also provided with means for introducing illumination 15 in the form of an exemplary light port or post 15 (that houses an optical fiber system or light guiding fiber bundles) and eyepiece 10 (viewer 10) or an alternate attachment to a camera (for example, a connector to a video viewing, or a direct camera interface such as a C-Mount). Exemplary light post 15 is positioned on the side of the handle and carries light from an external light source to the proximal end of the optical fibers and to the viewing end, where the light is emitted to illuminate the region of interest (i.e., the region that is inspected). Arthroscope 100 may further comprise fiber optic bundles, micro processors and cables, as known in the art.

Arthroscope 100 is also provided with reinforcement system 101 (reinforcement mechanism 101) which, according to an exemplary-only embodiment, comprises first and second reinforcement devices 30, 40 (first and second members or elements 30, 40) provided at respective first and second shaft locations or regions 25, 35 (reinforcing sections or regions 25, 35) on shaft 20.

The first reinforcement device 30 (first member 30) is a fluted tubular member having an outer diameter of about 6 mm, preferably about 6.2 mm, and is attached to the instrument 100 just distally to a means for introducing illumination 15 (for example, a light port 15 or light post 15) and at the most proximal portion of the arthroscopic "needle" 20. The first reinforcement device 30 completely surrounds shaft 20 at the first shaft region 25.

The second reinforcement device 40 (second member 40) is a tubular member having an outer diameter of about 5 mm, preferably about 5.2 mm, and is attached on the arthroscopic "needle" 20 except the most distal area 45 of the needle 20. The second reinforcement device 40 completely surrounds shaft 20 at the second shaft region 35.

The first and second reinforcement devices 30, 40 are adjacent each other (contact and abut each other) and extend in a direction about parallel to longitudinal axis 60 of the instrument 100, providing sufficient mechanical strength to an arthroscope (or similar instrument) such that a sheath is not needed. The first and second reinforcement devices 30, 40 are cost-effective devices that can attach to a conventional sheathless instrument (endoscope/arthroscope) and have a design that allow a smaller diameter for the instrument.

Fluted tube member 30 is a larger diameter tube provided at first reinforcing section 25 to provide reinforcement in the most highly stressed area. Tube 40 is smaller in diameter than fluted tube member 30 and is provided at the second tubular reinforcing section 35 (surrounding arthroscopic scope needle 20) to provide additional reinforcement.

Fluted tube reinforcing member 30 has an outer diameter (D1) of about 6 mm, preferably about 6.2 mm, at the most proximal portion of the arthroscopic "needle" 20. This reinforcing (reinforcement) device mechanically reinforces the fragile optics in the most highly stressed area. Fluted tube member 30 is provided with a plurality of flutes 33 which enable fluid flow when inserted into a cannula (such as cannula 200 of FIGS. 7 and 8).

Tubular reinforcement member 40 extends distally from the fluted member 30, and has an outer diameter (D2) of about 5 mm, preferably 5.2 mm, which provides mechanical support for all but the most distal area 45, which is not typically loaded during surgery. Distal area 45 has a length that is at least about 20% of the length of the needle extending distally from the distal end of fluted tube member 30, or is at least about 25% of the length of the needle extending distally from the distal end of fluted tube member 30, or is at least about 30% of the length of the needle extending distally from the distal end of fluted tube member 30, or about 35% of the length of the needle extending distally from the distal end of fluted tube member 30.

The inner diameters of reinforcing members 30 and 40 closely match that of the outer diameter of needle 20 such that members 30 and 40 can be slid over needle 20 and attached/fixed/bonded to the arthroscope needle 20 by welding, adhesion, or by any other suitable technique known in the art, depending on whether the reinforcing members are metallic or polymeric. Preferably, members 30 and 40 are manufactured from stainless steel and are covered with a dark, preferably black colored, thin coating 50, as is body 5. Coating 50 does not extend over distal section 45.

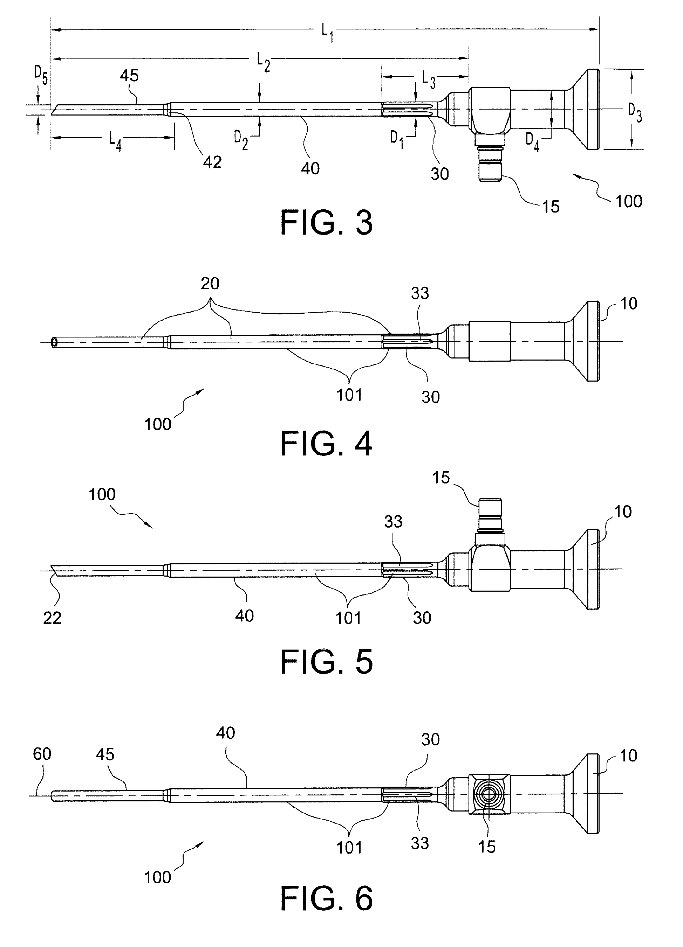

In an exemplary-only embodiment, arthroscope 100 of FIG. 3 provided with reinforcement system 101 has the following exemplary dimensions and specifications:
L1 = about 220-222 mm
L2 = about 168-169 mm
L3 = about 30-40 mm
L4 = about 50 mm
D1 = about 6-6.2 mm
D2 = about 5 mm
D3 = about 31-32 mm
D4 = about 15 mm
D5 = about 4 mm

As shown in FIGS. 7-9, exemplary arthroscopic system 300 of the invention includes arthroscope 100 provided with reinforcement system 101 and cannulas such as that disclosed in incorporated by reference U.S. Patent Publication No. 2003/0153926 and/or cannula 200 illustrated in FIGS. 7-9, which enable the system 300 to be used without a sheath due to the member reinforcement discussed above.

Exemplary cannula 200 of FIGS. 7-9 has a proximal end 205, an elongated shaft 220, and a distal end 210. A fluid passage 215 is located toward the proximal end 205. The fluid passage 215 is tubular and can be connected to a source of irrigation solution for supplying irrigation through the cannula to the work site during the arthroscopic procedure.

Although the light port (post) 15 is shown in FIGS. 1-3, 5 and 9 as extending about perpendicular to longitudinal axis 60 (central axis 60) of the shaft of the instrument, the invention is not limited to this exemplary-only embodiment and contemplates posts/ports or similar structures for introducing illumination to the instrument extending in a direction non-parallel to the longitudinal axis 60 of the instrument.

The reinforcement system 101 described above includes cost effective devices that can be attached to typical endoscopes/arthroscopes or similar instruments (manufactured in high volume and therefore more cost-effective) to enable use of the instrument without a sheath. The improved ergonomic design increases the efficiency of the arthroscopic procedure. The entire system can be permanently attached to the scope (instrument) or may be employed as a detachable, separately sterilizable sheath. If separable, a standard arthroscope could be used, enabling the use of a standard sheath or the "sheathless" system (reinforcement system 101) of the invention with the same arthroscope.

A method of endoscopic surgery with an endoscope or similar instrument provided with the mechanical reinforcement system 101 1 of the present invention comprises the steps of: (i) securing at least one element 30, 40 of a reinforcement system 101 to a shaft 20 of a sheathless endoscope 100, the reinforcement system 101 being provided just distally of a light post 15 (or similar means for introducing illumination) and surrounding the shaft of the instrument, to eliminate the use of a sheath of the instrument; and (ii) introducing the instrument with a cannulabut without an external or outer sheath -- at the arthroscopic site, to conduct at least one endoscopic/arthroscopic procedure.

While the present invention is described herein with reference to illustrative embodiments for particular applications, it should be understood that the invention is not limited thereto. Those having ordinary skill in the art and access to the teachings provided herein will recognize additional modifications, applications, embodiments and substitution of equivalents all falling within the invention. Accordingly, the invention is to be limited not by the specific disclosure herein, but only by the appended claims.

## Claims

1. An endoscope, comprising:
a shaft comprising a longitudinal axis, a proximal end and a distal end; and
a mechanical reinforcement device attached to the shaft and completely surrounding at least a portion of the shaft to eliminate the need for a sheath of the endoscope.

2. An endoscope according to claim 1, wherein the mechanical reinforcement device comprises a fluted tubular member located distally and adjacent a port located at the proximal end of the shaft, the port extending away from the shaft and in a direction non-parallel to the longitudinal axis of the shaft, the fluted tubular member comprising a plurality of flutes that extend radially from the longitudinal axis of the shaft and in a direction parallel to the longitudinal axis of the shaft, the plurality of flutes enabling fluid flow when the endoscope is inserted into a cannula.

3. An endoscope according to claim 2, wherein the mechanical reinforcement device further comprises a tube surrounding a region of the shaft located adjacent the fluted tubular member and distally to the fluted tubular member.

4. An endoscope according to claim 3, wherein the tube is in contact with the fluted tubular member and extends in a direction parallel to, and coaxial with, the fluted tubular member, wherein the tube has an outer diameter smaller than an outer diameter of the fluted tubular member.

5. An endoscope according to any one of claims 1 to 4, wherein the mechanical reinforcement device is formed of stainless steel and is covered by a coating.

6. An endoscope according to any one of claims 1 to 5, wherein the mechanical reinforcement device extends for more than two thirds of a length of the shaft.

7. An endoscope according to claim 1, wherein the mechanical reinforcement device comprises at least one of first and second elements, wherein the first element is a fluted tubular member provided with a plurality of flutes extending longitudinally relative to a central axis of the fluted tubular member, the first element completely surrounding a first region of the shaft and being adjacent and in contact with a post of the endoscope located at the proximal end of the shaft, the post extending away from the shaft and in a direction non-parallel to the longitudinal axis of the shaft, and wherein the second element is another tubular member with an outer diameter smaller than an outer diameter of the fluted tubular member, the second element extending coaxially with and distal to the first element and in contact with the first element.

8. An endoscope according to claim 7, wherein the outer diameter of the first element is about 6 mm and the outer diameter of the second element is about 5 mm.

9. An endoscope according to claim 7 or 8, wherein the first and second elements are integral with the endoscope.

10. An endoscope according to claim 7 or 8, wherein the first and second elements are removable from the endoscope.

11. An endoscope according to claim 7 or 8, wherein the first and second elements are slidable over the shaft of the endoscope.

12. An endoscope according to claim 7 or 8, wherein at least one of the first and second elements is attached to the shaft of the endoscope by bonding, snap-fitting, welding or adhesion.

13. An endoscope according to any one of claims 1 to 12, wherein the mechanical reinforcement device is a single-use disposable device.

14. An endoscope according to any one of claims 1 to 12, wherein the mechanical reinforcement device is removably attached to the shaft.

15. An endoscope according to any one of claims 1 to 12, wherein the mechanical reinforcement device is permanently attached to the shaft.
